# EUROPÄISCHE PATENTSCHRIFT

(11) **EP 3 110 470 B1**
(45) Veröffentlichungstag und Bekanntmachung des Hinweises auf die Patenterteilung: **20.10.2021**
(21) Anmeldenummer: 15707916.1
(22) Anmeldetag: 27.02.2015
(51) Int. Cl.: A61M 1/14, A61M 1/16, A61M 1/36

(54) **BLUTBEHANDLUNGSKASSETTE MIT STERILISATIONSÖFFNUNG UND ZUGEHÖRIGEM VERSCHLUSS SOWIE BLUTBEHANDLUNGSVORRICHTUNG**
BLOOD TREATMENT CASSETTE HAVING A STERILIZATION OPENING AND AN ASSOCIATED CLOSURE, AND BLOOD TREATMENT APPARATUS
CARTOUCHE DE TRAITEMENT DE SANG AYANT UNE OUVERTURE DE STÉRILISATION ET FERMETURE ASSOCIÉE AINSI QUE DISPOSITIF DE TRAITEMENT DE SANG

(30) Priorität: 27.02.2014 DE 102014102598
(43) Veröffentlichungstag der Anmeldung: 04.01.2017
(73) Patentinhaber: Fresenius Medical Care Deutschland GmbH, 61352 Bad Homburg (DE)
(72) Erfinder: HÄCKER, Jürgen, 61267 Neu-Anspach (DE)
(74) Vertreter: Bobbert & Partner Patentanwälte PartmbB
(86) Internationale Anmeldenummer: PCT/EP2015/054163
(87) Internationale Veröffentlichungsnummer: WO 2015/128474

(56) Entgegenhaltungen:
- WO-A1-2013/017235
- DE-A1-102009 024 469
- US-A1- 2009 012 450
- US-A1- 2011 064 608
- US-A1- 2013 138 037
- None

## Beschreibung

Die vorliegende Erfindung betrifft eine Blutbehandlungskassette gemäß dem Oberbegriff des Anspruchs 1 und eine Blutbehandlungsvorrichtung gemäß dem Oberbegriff des Anspruchs 4.

Einmalteilsysteme werden in der Medizin- oder Labortechnik zunehmend als kompakte medizinische Funktionsvorrichtungen wie Kassettensysteme oder Blutbehandlungskassetten ausgeführt, in denen Flüssigkeiten und Gase, insbesondere medizinische Flüssigkeiten und Blut, in Kanälen und Kammern geführt werden. Sind sie zum einmaligen Gebrauch vorgesehen, so spricht man von Kassettendisposables oder Einwegkassetten.

Die Blutbehandlungskassette wird vor ihrer Verwendung sterilisiert, beispielsweise mittels eines Sterilisationsfluids (Dampf, Flüssigkeit, Gas, usw.), welches die Blutbehandlungskassette durchströmt.

Bei der Dampfsterilisation der Blutbehandlungskassette, beispielsweise in einem Sterilisationsbehältnis eines Autoklaven, wird in mehreren Zyklen die im Sterilisationsbehältnis befindliche Luft vollständig durch Heißdampf ausgetauscht. Das Sterilisationsbehältnis mit der sich darin befindenden Blutbehandlungskassette (als Sterilistationsgut) wird mittels Vakuumpumpen evakuiert und anschließend mit Heißdampf bei Überdruck beströmt. Der Heißdampf kondensiert an den zu sterilisierenden Oberflächen der Blutbehandlungskassette. Dieser Vorgang wird je nach Größe und Beschaffenheit der Blutbehandlungskassette entsprechend oft wiederholt. Für die sterilisierende Wirkung ist es wichtig, dass an allen Oberflächen der Blutbehandlungskassette Dampf kondensiert. Weist die Blutbehandlungskassette Hohlräume bzw. Kavitäten mit flexiblen Elementen auf, kann es aufgrund der großen Druckwechsel während den einzelnen Sterilisationsphasen zu unerwünschten Verformungen kommen. Insbesondere eine Blutbehandlungskassette mit mittels Folie überspannten großen Kavitäten (wie Kammern oder Kanälen) eines Hartteils des Blutkassettenkörpers stellt diesbezüglich eine besondere Herausforderung dar. Die Strömungswege zu den Kavitäten weisen zum Teil Engstellen auf (wie Hydrophobmembranen oder lange, dünne Schläuche), die zur Folge haben, dass während der Druckwechsel im Innenraum der Kavitäten ein anderer Druck herrscht, als im Außenraum. Die während der Sterilisation zusätzlich herrschende hohe Temperatur führt dann unter Umständen zu irreversiblen Verformungen der Folie.

Eine Ventilvorrichtung, ein Ventileinsatz, eine externe Funktions- sowie Behandlungsvorrichtung und Verfahren sind aus der DE 10 2009 024 469 A1 bekannt.

Aus den WO 2013/017235 A1 ist eine Berührschutzvorrichtung für medizinische fluidführende Kassetten bekannt.

Ein mit Gas sterilisierbarer Dialysekonnektor und eine ebensolche Dialysekappe ist aus der US 2011/0064608 A1 bekannt.
Eine medizinische Maschine mit automatischer Versorgungsverbindung ist in US 2013/0138037 A1 offenbart.

Andere Sterilisationsmöglichkeiten sind aus der US 2009/0012450 A1 bekannt.

Eine erfindungsgemäße Aufgabe kann darin bestehen, eine weitere Blutbehandlungskassette mit einer Vorrichtung zum Verbessern der Sterilisierbarkeit der Blutbehandlungskassette vorzuschlagen. Ferner soll eine Blutbehandlungsvorrichtung zum Verwenden der Blutbehandlungskassette angegeben werden.

Die Aufgabe kann gelöst werden durch eine Blutbehandlungskassette mit den Merkmalen des Anspruchs 1, ferner durch eine Blutbehandlungsvorrichtung mit den Merkmalen des Anspruchs 4.

Erfindungsgemäß wird somit eine Blutbehandlungskassette vorgeschlagen, mit einem als Hartteil ausgestalteten Kassettenkörper. Die Blutbehandlungskassette kann optional eine Folie aufweisen. Ist eine Folie vorgesehen, so ist sie mit dem Hartteil verbunden und deckt das Hartteil im Gebrauch der Blutbehandlungskassette zumindest abschnittsweise ab.

Das Hartteil oder ein anderer Abschnitt der Blutbehandlungskassette weist wenigstens eine Sterilisationsöffnung auf. Diese weist wenigstens eine Verschlussvorrichtung, beispielsweise in Gestalt eines Verschlussstopfens, auf.

Ferner wird eine Blutbehandlungsvorrichtung vorgeschlagen, welche zur Verbindung mit einer erfindungsgemäßen Blutbehandlungskassette konfiguriert oder vorgesehen ist. Die Blutbehandlungsvorrichtung weist wenigstens einen, beispielsweise in einer Aktor-Sensor-Matte oder -Platte vorgesehenen, einteiligen oder mehrteiligen, Aktuator (hierin auch als Aktor bezeichnet) auf. Letzterer ist vorgesehen, ausgestaltet und/oder konfiguriert für eine Interaktion mit der Verschlussvorrichtung der Blutbehandlungskassette, um die Verschlussvorrichtung von der ersten Stellung in die zweite Stellung zu überführen. Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.
Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Hierin gemachte räumliche Angaben wie "oben", "unten", usw. beziehen sich im Zweifel auf die Darstellung, wie sie den hier beigefügten Figuren zu entnehmen ist.

Vorteilhafte Weiterentwicklungen der vorliegenden Erfindung sind jeweils Gegenstand von Unteransprüchen und Ausführungsformen.

Erfindungsgemäße Ausführungsformen können eines oder mehrere der im Folgenden genannten Merkmale in beliebiger Kombination aufweisen.

Erfindungsgemäß ist die Verschlussvorrichtung der Blutbehandlungskassette ausgestaltet, um in einem Zustand, in welchem sie mit der Sterilisationsöffnung verbunden ist, von einer ersten Stellung in eine zweite Stellung überführbar zu sein. Dabei gilt, dass die Verschlussvorrichtung in der ersten Stellung einen Fluidaustausch zwischen dem Inneren und einem Äußeren der Blutbehandlungskassette mittels der Sterilisationsöffnung (also durch letztere hindurch) zulässt, während sie in der zweiten Stellung einen solchen Fluidaustausch nicht zulässt. Die erste Stellung wird hierin auch als geöffnete Stellung bezeichnet, die zweite Stellung als geschlossene Stellung.

Erfindungsgemäß ist die Verschlussvorrichtung mit einem Mechanismus ausgestaltet, mittels welchem sie in der ersten und in der zweiten Stellung gehalten werden kann.

In bestimmten beispielhaften, erfindungsgemäßen Ausführungsformen ist der Mechanismus eine Verrastvorrichtung oder weist eine solche auf.

In einigen beispielhaften, erfindungsgemäßen Ausführungsformen der Blutbehandlungskassette ist die Verschlussvorrichtung ausgestaltet, um auch nach erfolgtem Sterilisieren der Blutbehandlungskassette in die zweite Stellung übergeführt zu werden, selbst wenn die Blutbehandlungskassette dabei in einer die Sterilität schützenden Hülle oder Verpackung vorliegt. Die Verschlussvorrichtung kann in solchen Ausführungsformen manuell und/oder - beispielsweise mittels geeigneter Mechanik - automatisch in die zweite Stellung übergeführt werden.

In bestimmten beispielhaften, erfindungsgemäßen Ausführungsformen ist die Sterilisationsöffnung im Hartteil vorgesehen oder ausgestaltet.

In manchen beispielhaften, erfindungsgemäßen Ausführungsformen weist die Verschlussvorrichtung wenigstens zwei, vorzugsweise getrennt voneinander vorliegende Dichtflächenpaarungen, Dichtelemente oder -abschnitte auf.

In bestimmten beispielhaften, erfindungsgemäßen Ausführungsformen ist die Blutbehandlungsvorrichtung mit einer erfindungsgemäßen Blutbehandlungskassette verbunden.

In manchen beispielhaften, erfindungsgemäßen Ausführungsformen ist der Aktuator eine unveränderbare Erhebung in oder an einer Aktor-Sensor-Matte, einer Aktor-Sensor-Platte oder einer Tür der Blutbehandlungsvorrichtung. Dabei ist die Erhebung beispielsweise in ihrer Höhe, ihrer Erhebung über dem Niveau seiner direkten Umgebung, seiner Geometrie oder dergleichen unveränderbar.

In bestimmten beispielhaften, erfindungsgemäßen Ausführungsformen weist die Blutbehandlungsvorrichtung eine Vorrichtung auf, welche konfiguriert ist, den Aktuator oder eine seiner Eigenschaften, wie etwa seine Höhe, seine Erhebung relativ zum Niveau seiner direkten Umgebung, seine Geometrie oder dergleichen, zu verändern und/oder die Verschlussvorrichtung mittels des Aktuators von der ersten Stellung in die zweite Stellung zu überführen.

In manchen beispielhaften, erfindungsgemäßen Ausführungsformen weist die Blutbehandlungsvorrichtung eine Steuervorrichtung und wenigstens einen Sensor auf. Dabei ist die Blutbehandlungsvorrichtung beispielsweise mittels ihrer Steuervorrichtung oder eines anderen Abschnitts konfiguriert, um mittels des Sensors festzustellen, ob sich die Verschlussvorrichtung in der ersten oder der zweiten Stellung befindet. Ferner ist die Blutbehandlungsvorrichtung - beispielsweise wiederum mittels der Steuervorrichtung oder eines anderen Abschnitts konfiguriert, um eine Blutbehandlung mittels der Blutbehandlungsvorrichtung zu unterbinden oder zu unterbrechen, sollte festgestellt worden sein, dass sich die Verschlussvorrichtung nicht in der zweiten Stellung befindet. Alternativ ist sie konfiguriert, um eine Blutbehandlung nur zuzulassen, wenn festgestellt wurde, dass die Verschlussvorrichtung in der ersten Stellung steht.

In bestimmten beispielhaften, erfindungsgemäßen Ausführungsformen dient die Sterilisationsöffnung allein dem Sterilisationsprozess.

In manchen beispielhaften erfindungsgemäßen Ausführungsformen ist und bleibt die Sterilisationsöffnung nach Abschluss des Sterilisationsprozesses, vorzugsweise irreversibel oder bis zur Aufbringung von zerstörerischen Kräften, geschlossen.

In einigen bevorzugten Ausführungsformen ist die Sterilisationsöffnung keine Single-Needle-Sterilmembran und/oder keine Aufnahme im Hartteil dafür.

In einer weiteren Ausführungsform weisen die Kassette oder deren Hartteil wenigstens eine Single-Needle-Sterilmembran und zusätzlich wenigstens eine erfindungsgemäße Sterilisationsöffnung auf.

In manchen beispielhaften erfindungsgemäßen Ausführungsformen sind Single-Needle-Sterilmembran und Sterilisationsöffnung unterschiedliche und voneinander getrennte Strukturen, welche sich in unterschiedlichen Abschnitten der Kassette befinden können.

In manchen beispielhaften erfindungsgemäßen Ausführungsformen sind Dichtflächenpaare Kombinationen aus zwei vergleichsweise harten Materialien. In anderen beispielhaften erfindungsgemäßen Ausführungsformen ist wenigstens eine dieser Flächen elastisch.

In manchen beispielhaften erfindungsgemäßen Ausführungsformen ist der Aktuator ausgestaltet, um passiv auf die Verschlussvorrichtung einzuwirken, in anderen, um aktiv auf die Verschlussvorrichtung einzuwirken. Bei einer passiven Ausgestaltung kann der Aktuator beispielsweise als feststehende, maschinenseitige Nocke oder Erhebung ausgestaltet sein Bei einer aktiven Ausgestaltung kann der Aktuator beispielsweise als verfahrbarer, maschinenseitiger Stößel ausgestaltet sein.

In bestimmten erfindungsgemäßen Ausführungsformen ist die Blutbehandlungskassette ein Disposable oder ein Einmalartikel.

In bestimmten erfindungsgemäßen Ausführungsformen sind die Blutbehandlungskassette und/oder die Blutbehandlungsvorrichtung zur Apherese, zur Hämodialyse, zur Hämofiltration, Hämodiafiltration, Hämoultrafiltration und dergleichen konfiguriert.

Einige oder alle erfindungsgemäßen Ausführungsformen können einen oder mehrere der oben oder im Folgenden genannten Vorteile aufweisen.

Zu den erzielbaren Vorteilen kann zählen, dass es trotz der großen Druckwechsel während der einzelnen Sterilisationsphasen nicht zu unerwünschten Verformungen der Hohlräume bzw. Kavitäten mit flexiblen Elementen der Blutbehandlungskassette kommt.

Von Vorteil kann ferner sein, dass die zusätzlich herrschende hohe Temperatur während der Sterilisation, die u. a. durch die herrschenden Druckwechsel im Innen- und Außenraum bedingt ist, selbst bei Folien-Kassetten nicht zu irreversiblen Verformungen selbst jener Folienabschnitte führt, welche große Kavitäten, wie Kammern oder Kanäle eines Hartteils des Blutkassettenkörpers überspannen.

Ein weiterer Vorteil kann sein, dass eine bleibende Verformung der Folie, sofern vorhanden, verhindert wird, indem der Druckausgleich zwischen dem Innenraum der Kavitäten und dem Außenraum über die Sterilisationsöffnung hinweg beschleunigt wird.

Die vorliegende Erfindung wird im Folgenden anhand der beigefügten Zeichnung, in welcher identische Bezugszeichen gleiche oder ähnliche Bauteile bezeichnen, exemplarisch erläutert. In den zum Teil vereinfachten Figuren gilt:
- **Fig. 1**: zeigt eine Seitenansicht einer an ihrer Vorderseite mit einer Abdeckungseinrichtung versehenen erfindungsgemäßen Blutbehandlungskassette gemäß einer bevorzugten Ausführungsform;
- **Fig. 2**: zeigt die Blutbehandlungskassette der Fig. 1 mit nach zerstörendem Aufschneiden aufgeklappter Abdeckungseinrichtung;
- **Fig. 3**: zeigt schematisch vereinfacht einen Abschnitt des Hartteils der erfindungsgemäßen Blutbehandlungskassette, hierin auch als Kassette bezeichnet, mit der Sterilisationsöffnung und einer hierin vorgesehenen Verschlussvorrichtung in der ersten, offenen Stellung, im Längsschnitt;

- **Fig. 4**: zeigt die Sterilisationsöffnung mit der Verschlussvorrichtung der Fig. 3 in der zweiten, geschlossenen Stellung;
- **Fig. 5**: zeigt die Verschlussvorrichtung der Fig. 3 und 4 in perspektivischer Ansicht; und
- **Fig. 6**: zeigt eine Aktor-Sensor-Matte einer Aktor-Sensor-Platte und eine Maschinentür eines schematisch vereinfachten Ausschnitts einer erfindungsgemäßen Blutbehandlungsvorrichtung.

Die normalen Pfeile in den Figuren zeigen die Richtung des Blutstroms an. Die Blockpfeile zeigen jeweils die Richtung des Substituatstroms an.

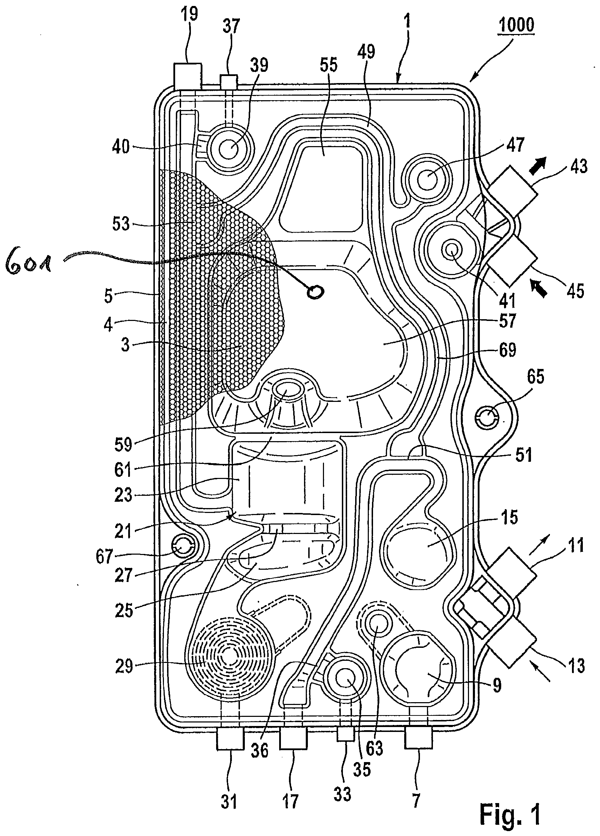

**Fig. 1** zeigt eine Seitenansicht einer erfindungsgemäßen Blutbehandlungskassette 1000, welche an der Oberfläche, auf die in Fig. 1 geblickt wird, mit einer Abdeckungseinrichtung versehen ist.

Die erfindungsgemäße Blutbehandlungskassette 1000 wird im Folgenden auch kurz als Kassette 1000 bezeichnet.

Die Kassette 1000 weist ein Hartteil 1 auf. Wie in Fig. 1 exemplarisch gezeigt, weist das Hartteil 1 Kammern, Kanäle und Ventile auf. Wie in Fig. 1 exemplarisch weiter gezeigt ist, sind die Kammern, Kanäle und Ventile in das Hartteil 1 integriert bzw. zumindest teilweise vom Hartteil 1 ausgestaltet.

Die Kassette 1000 der Fig. 1 ist an ihrer Vorderseite mit einer Abdeckungseinrichtung, hier beispielsweise einer Folie 3, versehen. Die Abdeckungseinrichtung kann eben, d.h. plan, auf das Hartteil 1 aufgeschweißt sein.

Eine Ausgestaltung mit einer dreidimensionalen Ausführung der Schweiß- und Dichtkontur ist erfindungsgemäß ebenfalls möglich.

Die Abdeckungseinrichtung kann die Kammern und/oder Kanäle des Hartteils 1 der Kassette 1000 verschließen, und zwar gegenüber einer dem Hartteil 1 abgewandten Seite der Abdeckungseinrichtung und/oder gegenüber der Atmosphäre.

Wie in Fig. 1 zu erkennen ist, liegt die Folie an einem umlaufenden Dichtsteg 4 auf dem Hartteil 1 der Kassette 1000 auf. Die Folie 3 ist an einer umlaufenden Schweißnaht 5 mit dem Hartteil 1 der Kassette 1000 verschweißt.

Der umlaufende Dichtsteg 4 kann alternativ freiliegend ausgeführt werden.

Die Folie 3 kann an weiteren lokalen Verschweißungen (nicht gezeigt) mit dem Hartteil 1 der Kassette 1000 verbunden sein. Diese können ebenfalls umlaufend, also geschlossen im Sinne einer abschließenden Begrenzung ähnlich einem Ring, und/oder punktförmig sein.

Die Folie 3 kann an lokalen Stellen punkt- oder linienförmig mit dem Hartteil der Kassette 1000 verbunden, z. B. verschweißt, sein, insbesondere an den Randzonen der flüssigkeitsführenden Kanäle.

Die Folie 3 kann durch Laser-Schweißen mit dem Hartteil der Kassette 1000 verbunden werden. Dabei ist es vorteilhaft, wenn der lokale Hitzeeintrag unter Verwendung einer Licht absorbierenden Komponente erfolgt. Die Licht absorbierende Komponente kann Bestandteil des Materials der Folie und/oder des Hartteils sein oder eine Schicht, die zwischen Folie und Hartteil oder über der Folie angeordnet ist. Die Schicht kann eine Folienschicht sein.

Die Kassette 1000 kann wenigstens mit der in Fig. 1 gezeigten Vorderseite an eine Blutbehandlungsvorrichtung (in Fig. 1 nicht gezeigt) angekoppelt werden. Eine beispielhafte Vorgehensweise zum geeigneten Ankoppeln einer Kassette 1 an eine Ankoppelfläche einer Blutbehandlungsvorrichtung ist in den Patentanmeldungen 10 2009 012 633.3 mit dem Titel *"Vorrichtung zum Verbinden einer externen Funktionseinrichtung mit einer Anordnung, Anordnung aufweisend eine solche Vorrichtung und Verfahren zum Verbinden",* die am 10. März 2009 beim DPMA eingereicht wurde, und 10 2009 012 632.5 mit dem Titel *"Abdichtungseinrichtung zum Abdichten eines Volumens einer medizinischen Behandlungsanordnung gegen ein weiteres Volumen sowie Anordnung und Verfahren",* die ebenfalls am 10. März 2009 beim DPMA eingereicht wurde, beschrieben.

Die Kassette 1000 kann mit der Ebene der Folie 3 - oder über diese - an eine Ankoppelfläche der Blutbehandlungsvorrichtung angekoppelt werden.

Die Ankoppelfläche der Blutbehandlungsvorrichtung kann zum Beispiel an einem in Fig. 1 oberen Abschnitt hiervon um 8° gegen eine in Fig. 1 von oben nach unten verlaufende Senkrechte nach hinten (in der Fig. 1 die sich vom Betrachter in die Zeichenebene hinein erstreckende Richtung) geneigt sein.

Die Kassette 1000 weist einen arteriellen Patientenanschluss 7 auf.

Die Kassette 1000 weist einen Konnektor 11 für den Blutaustritt aus der Kassette 1000 sowie einen Konnektor 13 für den Bluteintritt in die Kassette 1000 auf.
Die beiden Konnektoren 11 und 13 sind mit einem Pumpschlauchsegment oder -set einer Blutpumpe verbindbar.

Die Kassette 1000 weist ferner eine Kammer 15 mit einer Druckmessstelle zur Druckmessung im extrakorporalen Blutkreislauf vor dem Dialysator ("Prä-Filter") bzw. nach der Pumpe ("Post-Pumpe") auf.

An der Kammer 15 kann durch die Folie 3 hindurch bzw. über die Folie 3 der Druck im extrakorporalen Kreislauf vor dem Dialysator gemessen werden.

Die Kassette 1000 weist eine arterielle Filterleitung 17 sowie eine venöse Filterleitung 19 auf.

Das Innere der Kassette 1000 weist eine venöse Blutkammer 21 auf. Die venöse Blutkammer 21 ist in einen oberen Raum 23 und einen unteren Raum 25 unterteilt.

Der obere Raum 23 der venösen Blutkammer 21 kann eine seitlich tangentiale Bluteinströmung zulassen. Dabei kann Blut seitlich durch den Einlass (der linken Seite in Fig. 1) in den oberen Raum 23 einströmen und sich tangential zu den Wänden des oberen Raums 23 ausbreiten. Eine seitlich tangentiale Bluteinströmung kann eine Zone mit im Wesentlichen oder vollständig stabiler Rotationsströmung des Bluts in dem oberen Raum 23 der venösen Blutkammer 21 erzeugen.

Der untere Raum 25 der venösen Blutkammer 21 kann eine Beruhigungszone für die Blutströmung darstellen. Es ist möglich, dass in einer derartigen Beruhigungszone im Wesentlichen keine oder überhaupt keine Rotationsströmung des Bluts vorliegt.

Die venöse Blutkammer 21 ist durch eine Querschnittsverjüngung 27 des Hartteils 1 der Kassette 1000 in den oberen Raum 23 und den unteren Raum 25 aufgeteilt. Die Querschnittsverjüngung 27 reduziert den Querschnitt der venösen Blutkammer 21 derart in seiner Breite und Tiefe, dass sich eine Strömungsschnelle ergibt, nach deren Durchquerung, ein die venöse Blutkammer 21 der Kassette 1000 durchströmendes, Fluid eine langsamere Strömungsgeschwindigkeit annimmt. Der obere Raum 23 und der untere Raum 25 stehen in Fluidkommunikation.

Durch eine derartige Konstruktion, d.h. einer Aufteilung der venösen Blutkammer 21 in eine Zone mit im Wesentlichen oder vollständig stabiler Rotationsströmung des Bluts und in eine Beruhigungszone für die Blutströmung, kann vorteilhaft eine effiziente Abscheidung für Luft aus dem Blut oder Fluid erreicht werden.

Wandungen des oberen Raums 23 und des unteren Raums 25 der venösen Blutkammer 21 können in geeigneter Weise einer Neigung des oberen Abschnitts der Kassette 1000 in Fig. 1 gegen die Vertikale, beispielsweise einer Neigung des oberen Teils der in Fig. 1 gezeigten Kassette 1000 um 8° nach hinten (in die Zeichenebene hinein), angepasst werden. Sie können in geeigneter Weise derart gerundet ausgestaltet sein, dass sie vorteilhaft eine strömungsoptimierte Berührfläche für Fluide darstellen, welche die venöse Blutkammer 21 durchströmen.

Die Kassette 1000 weist einen Gerinnselfänger 29 auf. Als Gerinnselfänger kann vorzugsweise ein Gerinnselfänger verwendet werden, wie er in der von der Anmelderin der vorliegenden Erfindung in der Patentanmeldung 10 2009 024 495.6 mit dem Titel "Gerinnselfänger, externe Funktionseinrichtung, Blutkreislauf sowie Behandlungsvorrichtung", die am 10. Juni 2009 beim DPMA eingereicht wurde, offenbart wurde.

Am Gerinnselfänger 29 kann durch die Folie 3 hindurch bzw. über die Folie 3 der Druck im extrakorporalen Kreislauf gemessen werden, also insbesondere nach Durchlaufen des Dialysators.

Die Kassette 1000 weist einen venösen Patientenanschluss 31 auf.

Die Kassette 1000 weist eine arterielle Heparin-Zugabestelle 33 auf. Dabei gilt zu beachten, dass die Heparin-Zugabestelle 33 (wie auch eine venöse Heparin-Zugabestelle 37) auch zur Zugabe anderer pharmakologischer Wirkstoffe als Heparin, welche nur vorzugsweise Antikoagulantien sind, oder Wirkstoffkombinationen geeignet und vorgesehen sein kann. Dies ist stets auch dann zu beachten, wenn zuvor oder im Folgenden von Heparin in beliebigem Zusammenhang die Rede ist.

Die Kassette 1000 weist ein Rückschlagventil 35 der arteriellen Heparin-Zugabestelle 33 auf.

Beispielhafte Rückschlagventile zum Einsatz als Rückschlagventil 35 der arteriellen Heparin-Zugabestelle 33 sowie als weitere Rückschlagventile der Kassette 1000 sind in der Patentanmeldung 10 2009 024 469.7 der Anmelderin der vorliegenden Erfindung mit dem Titel "Ventilvorrichtung, Ventileinsatz, externe Funktionseinrichtung, Behandlungsvorrichtung sowie Verfahren", die am 10. Juni 2009 beim DPMA eingereicht wurde, offenbart.

Die Kassette 1000 weist ein arterielles Heparin-Zugabeventil 36 auf. Mit Hilfe des arteriellen Heparin-Zugabeventils 36 kann die Zugabe von Heparin in die arterielle Filterleitung 17 gesteuert oder geregelt werden.

Das arterielle Heparin-Zugabeventil 36 kann als so genanntes Phantomventil ausgestaltet sein.

Der Begriff "Phantomventil", wie er hierin verwendet wird, bezeichnet ein Element mit einer mittels Aktor erreichbaren Aktor-Fläche (hier beispielsweise eine Aktor-Membran), welches die Funktion eines Ventils übernehmen kann.

Die Aktor-Membran ist unter Aufbringung einer Kraft hierauf, z.B. einer Druckkraft, in eine Richtung bewegbar, ausdehnbar bzw. wölbbar oder dergleichen. Durch das Bewegen oder Ausdehnen der Aktor-Membran kann sich diese an ein Element, wie eine Abdichtungseinrichtung, etwa einen Steg, anlegen oder von diesem entfernen. Die Aktor-Membran kann somit beispielsweise eine Abdichtung bewirken bzw. verstärken oder beenden bzw. verringern.

Wenn die Kraft wieder von der Aktor-Membran genommen wird, kann diese in beispielsweise eine Grundposition, z. B. einen nicht gewölbten Zustand, zurückkehren.

Ein Phantomventil zur Verwendung als arterielles Heparin-Zugabeventil 36 sowie weitere Phantomventile der Kassette 1000 können mit oder aus einem Stegabschnitt eines Kanals am Hartteil 1 der Kassette 1000 und einem dem Stegabschnitt an- oder gegenüberliegenden Abschnitt der Folie 3 ausgestaltet werden.

Phantomventile können durch Aktoren der Blutbehandlungsvorrichtung betätigt werden.

Zum Schließen eines Phantomventils kann der Abschnitt der Folie 3 auf den Stegabschnitt gedrückt werden. Zum Öffnen des Phantomventils kann der Abschnitt der Folie 3 wieder vom Stegabschnitt abgehoben werden.

Weitere Beispiele und/oder Ausführungsformen für Phantomventile können der Patentanmeldung 10 2009 012 632.5 mit dem Titel "Abdichtungseinrichtung zum Abdichten eines Volumens einer medizinischen Behandlungsanordnung gegen ein weiteres Volumen sowie Anordnung und Verfahren", die am 10. März 2009 von der vorliegenden Anmelderin beim DPMA eingereicht wurde, sowie der DE 100 53 441 A1 und der DE 102 24 750 A1 entnommen werden.

Die Kassette 1000 weist eine venöse Heparin-Zugabestelle 37 auf. Die venöse Heparin-Zugabestelle 37 kann als Luer-Konnektor ausgestaltet sein.

Die Kassette 1000 weist ein Rückschlagventil 39 der venösen Heparin-Zugabestelle 37 auf.

Die Kassette 1000 weist ein venöses Heparin-Zugabeventil 40 auf. Mit Hilfe des venösen Heparin-Zugabeventils 40 kann die Zugabe von Heparin in die venöse Filterleitung 19 gesteuert oder geregelt werden.

Die Kassette 1000 weist eine Substituat-Zugabestelle 41 bzw. einen Substituatkonnektor auf.

Die Substituat-Zugabestelle 41 kann eine Verbindungseinrichtung sein, wie sie in der Patentanmeldung 10 2009 024 575.8 der vorliegenden Anmelderin beschrieben ist, welche mit dem Titel *"Verbindungseinrichtung und Verfahren zum Konnektieren wenigstens zweier fluidführender medizintechnischer Systeme, sowie medizintechnische Vorrichtung"* am 10. Juni 2009 von der vorliegenden Anmelderin beim DPMA eingereicht wurde.

Die Substituat-Zugabestelle 41 kann mit einem Berührschutzelement (nicht gezeigt) versehen sein. Die Substituat-Zugabestelle 41 kann mit einem Tropfschutz (nicht gezeigt) versehen sein. Der Tropfschutz kann durch eine integrierte Verschlusshülse realisiert werden. Der Tropfschutz kann ein Heraustropfen von Resten von Substituat und/oder Blut beim Lösen der Kassette 1000 und anschließendem Entnehmen der Kassette 1000 aus der Blutbehandlungsvorrichtung verhindern.

Der Tropfschutz kann abnehmbar ausgeführt sein. Er kann als Haube oder Deckel ausgestaltet sein.

Die Substituat-Zugabestelle 41 oder ein anderer Abschnitt der Kassette 1000 kann ferner einen Originalitätsschutz bieten, durch welchen der Anwender ohne Mühe oder auf einen Blick erkennt, ob die Kassette 1000 bereits benutzt wurde. Dieser Originalitätsschutz kann mittels des Berührschutzelements, der Verschlusshülse oder einer anderen Struktur realisiert sein. Die entsprechende Struktur kann vorzugsweise ihre Position innerhalb der oder bezogen auf die Kassette 1000 erkennbar verändern. Sie kann vorzugsweise ihre Gestalt verändern.

Zudem kann die Substituat-Zugabestelle 41 oder ein anderer Abschnitt der Kassette 1000 einen Wiederverwendungsschutz bzw. Schutz gegen ein Wiederverwenden bieten. Vorzugsweise durch eine Verschlusshülse wird die Kassette 1000 - vorzugsweise irreversibel - im Hinblick auf den Versuch einer Wiederverwendung unbrauchbar gemacht. Soll die Kassette 1000 trotzdem erneut verwendet werden, so messen Sensoren der Blutbehandlungsvorrichtung nicht die Signalverläufe, die bei Verwenden einer neuen Kassette gemessen werden würden. Dies kann hierauf beruhen, dass keine Flüssigkeit in die Kassette 1000 oder in die Substituat-Zugabestelle 41 gelangen kann oder wenigstens nicht in ausreichender oder üblicher Menge. Die Steuereinheit der Blutbehandlungsvorrichtung kann dies erkennen. Es kann eine Warnung veranlasst werden.

Als Originalitätsschutz oder Wiederverwendungsschutz kann vorzugsweise ein Originalitätsschutz oder ein Wiederverwendungsschutz verwendet werden, wie er in der von der Anmelderin der vorliegenden Erfindung in der Patentanmeldung 10 2009 024 575.8 mit dem Titel "Verbindungseinrichtung und Verfahren zum Konnektieren wenigstens zweier fluidführender medizintechnischer Systeme, sowie medizintechnische Vorrichtung", die am 10. Juni 2009 beim DPMA eingereicht wurde, offenbart wurde.

Die Kassette weist einen Konnektor 43 für einen Substituataustritt aus der Kassette 1000 sowie einen Konnektor 45 für einen Sustituateintritt in die Kassette 1000 auf.

Die Konnektoren 43 und 45 sind mit einem Pumpschlauchsegment oder -set einer Substituatpumpe verbindbar.

Die Kassette 1000 weist ein Rückschlagventil 47 für Substituatzugabe auf.

Über Betätigung des Rückschlagventils 47 kann Substituat in eine Substituatleitung 49 eingebracht werden.

Die Kassette 1000 weist ein Prädilutions-Zugabeventil 51 auf. Das Prädilutions-Zugabeventil 51 kann als Phantomventil ausgestaltet sein.

Die Kassette 1000 weist ein Postdilutions-Zugabeventil 53 auf. Das Postdilutions-Zugabeventil 53 kann als Phantomventil ausgestaltet sein.

Die Kassette 1000 weist eine Single-Needle-Sterilmembran 55 auf.

Die Kassette 1000 weist eine Single-Needle-Kammer 57 auf. Die Single-Needle-Kammer 57 ist in Fig. 1 oberhalb der venösen Blutkammer 21 angeordnet.

Im Inneren der Single-Needle-Kammer 57 ist ein optionales Blutschwallumleitungselement 59 angeordnet. Das Blutschwallumleitungselement 59 kann dem Abbremsen eines Blutschwalls und/oder dem Auslöschen dessen Impulses dienen.

Eine Verbindung mit einem Inneren der Single-Needle-Kammer 57 kann mittels einer Verbindungseinrichtung vorgesehen sein, wie sie in der von der Anmelderin der vorliegenden Erfindung mit dem Titel *"Einrichtung sowie externe Funktionseinrichtung und Behandlungsvorrichtung zum Behandeln von medizinischen Fluiden"* 10 2009 024 467.0 am 10. Juni 2009 beim DPMA eingereichten Patentanmeldung offenbart wurde.

Die Kassette 1000 weist ein Single-Needle-Blutventil 61 auf. Das Single-Needle-Blutventil 61 kann als Phantomventil ausgestaltet sein.

Die Kassette 1000 weist eine Absaugstelle 63 auf. Die Absaugstelle 63 kann zur Vakuumankopplung der Kassette 1000 an die Blutbehandlungsvorrichtung dienen, wie dies beispielsweise in der Patentanmeldung DE 10 2007 042 964 A1 mit dem Titel "Vorrichtung und Verfahren zur Behandlung einer medizinischen Flüssigkeit", die am 10. September 2007 beim DPMA eingereicht wurde, beschrieben ist.

Die Kassette 1000 weist ein primäres Ausrichtungszentrum 65 auf. Das primäre Ausrichtungszentrum 65 kann vorteilhaft zum Ausrichten und/oder Verrasten der Kassette 1000 an der Blutbehandlungsvorrichtung dienen.

Die Kassette 1000 weist eine sekundäre Ausrichtungsstelle 67 auf. Die sekundäre Ausrichtungsstelle 67 kann zum Ausrichten und/oder Verrasten der Kassette 1000 an der Blutbehandlungsvorrichtung dienen.

Die Kassette 1000 ist vor Beginn des Primens mit Gas (z.B. steriler Luft) gefüllt. Beim Primen des extrakorporalen Blutkreislaufs muss diese Gasfüllung verdrängt werden. Eine Blutbehandlungskassette stellt hierbei allgemein eine besondere Herausforderung dar, weil es sowohl aufsteigende als auch fallende Leitungen und zudem Kammern gibt, in denen keine "Luftnester" verbleiben dürfen. Die vorliegende Kassette 1000 weist zu diesem Zweck besondere Konstruktionsmerkmale auf:
Die Kammer 15 zum Messen des arteriellen Drucks ist derart konstruiert, dass die gesamte Luft in ein Pumpschlauchsegment (z. B. in das Pumpschlauchsegment 90) aufsteigen kann. Es gibt hierbei vorteilhaft keine Toträume. Luft, die aus der arteriellen Druckmesskammer in das Pumpschlauchsegment der Blutpumpe selbständig aufsteigt, wird ab dem Eingriffsbereich der Blutpumpe (z.B. durch die Rollen einer Rollenpumpe) durch das Pumpschlauchsegment zwangsgefördert. Sobald die Pumpe keinen Einfluss mehr hat (weil z. B. die Rollen in Ausgriff gehen), steigt die Luft selbsttätig in Förderrichtung in die Kassette 1000 auf.

Die venöse Rückführleitung (bzw. ein venöser Abschnitt 93 des extrakorporalen Kreislaufs) ist eine Fallleitung. Ab einem darin herrschenden, gewissen Volumenstrom (z. B. 200 ml/min bei der in Fig. 1 gezeigten Kassette 1000) werden Luftblasen im Blut auch entgegen der Erdbeschleunigung "mitgerissen". Dieser Effekt wird bei den Fallleitungen ausgenutzt. Die Leitungsquerschnitte der Fallleitungen sind so klein ausgelegt, dass durch die Strömungsgeschwindigkeit eine Zwangsförderung der Luftblasen auch entgegen der Erdbeschleunigung funktioniert.

In der venösen Blutkammer 21 sind große Querschnitte vorgesehen, derart, dass aufgrund der dort langsamen bzw. niedrigen Strömungsgeschwindigkeiten Luftblasen verlässlich entgegen der Hauptströmungsrichtung aufsteigen können.

Weitere konstruktive Besonderheiten der Kassette 1000 sind:
Die Phantomventile 40, 51 und 53 sind räumlich derart ausgerichtet, dass Blut (welches eine höhere Dichte als Wasser bzw. Substituat etc. hat) bei Betrieb der Kassette 1000 mit Blut kaum "nach oben" oder "zur Seite" in geöffnete Phantomventile eindringen kann, weil es gegenüber dem leichteren Wasser absinkt. Eine solch vorteilhafte Ausrichtung ist durch die Phantomventile 40, 51, und 53 realisiert. Beim Ventil 36 besteht hingegen kein solches Erfordernis, d. h. dort kommt es auf die Orientierung nicht an.

Der Leitungskanal (Stichkanal) unter dem Rückschlagventil 47 für Substituatzugabe ist aus demselben Grunde aufsteigend konstruiert. Im Falle einer Fehlfunktion der Prä- und/oder Postdilutionsventile 51 und 53 und einer daraus resultierenden Blut-Bypassströmung kann kein Blut in die Substituatleitung 49 aufsteigen. Vielmehr strömt das Blut an der Mündung der entsprechenden Stichleitung vorbei.

Die Neigung der Kassette 1000 beträgt vorzugsweise 5° bis 11°, besonders bevorzugt die bereits genannten 8°.

Das Bezugszeichen 601 bezeichnet eine Sterilisationsöffnung. Sie verbindet ein Äußeres der Kassette 1000 mit einem Inneren hiervon. Die Sterilisationsöffnung 601 kann beispielsweise angeordnet sein, um ein Inneres der Single-Needle-Kammer 57 mit dem Äußeren zu verbinden. Sie kann in der Single-Needle-Kammer 57 angeordnet sein.

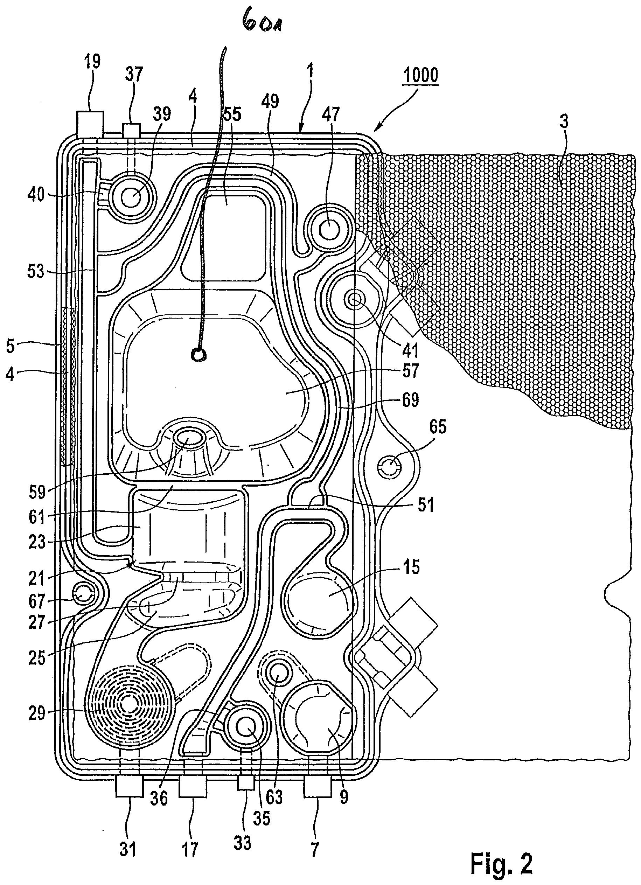

**Fig. 2** zeigt die Kassette 1000 der Fig. 1, wobei die Folie 3 am linken Rand der Kassette 1000 sowie oben und unten zur besseren Illustration zerstörend aufgeschnitten und nach rechts aufgeklappt zu erkennen ist.

Wie in Fig. 2 gezeigt, weist die Folie 3 eine Oberflächenstrukturierung auf.

Fig. 2 zeigt die nach Aufschneiden der Folie 3 detaillierter zu erkennenden Elemente im Inneren der Kassette 1000.

Zur Vermeidung von Wiederholungen wird auf die vorstehend bei der Beschreibung der Fig. 1 erörterten Ausgestaltungen der einzelnen Elemente verwiesen.

Gut zu erkennen ist hierbei, dass die Kassette 1000 einen Dichtsteg 69 aufweist. Der Dichtsteg 69 kann zum Beispiel zum Ausgestalten des Prädilutions-Zugabeventils 51 eingesetzt werden.

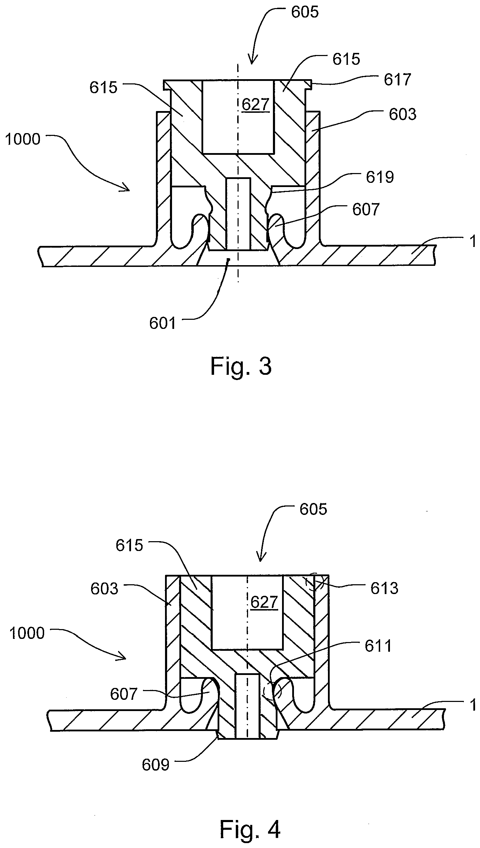

**Fig. 3** zeigt schematisch vereinfacht einen Abschnitt des Hartteils 1 der erfindungsgemäßen Blutbehandlungskassette 1000, hierin auch als Kassette 1000 bezeichnet, im Längsschnitt.

Das Hartteil 1 weist rein optional einen Zylinderabschnitt 603 auf, welcher die Sterilisationsöffnung 601 umgibt, sich zylinderartig an diese anschließt und/oder diese fluiddicht verlängert.

In den Zylinderabschnitt 603 ist, in Fig. 3 erkennbar, eine Verschlussvorrichtung 605 eingesetzt. Die Verschlussvorrichtung 605 ist ausgestaltet, um die Sterilisationsöffnung 601 verschließen zu können, in Abhängigkeit von der Stellung, die die Verschlussvorrichtung 605 bezogen auf die Sterilisationsöffnung 601 einnimmt und/oder in Abhängigkeit von der Verformung, die die Verschlussvorrichtung 605 annimmt.

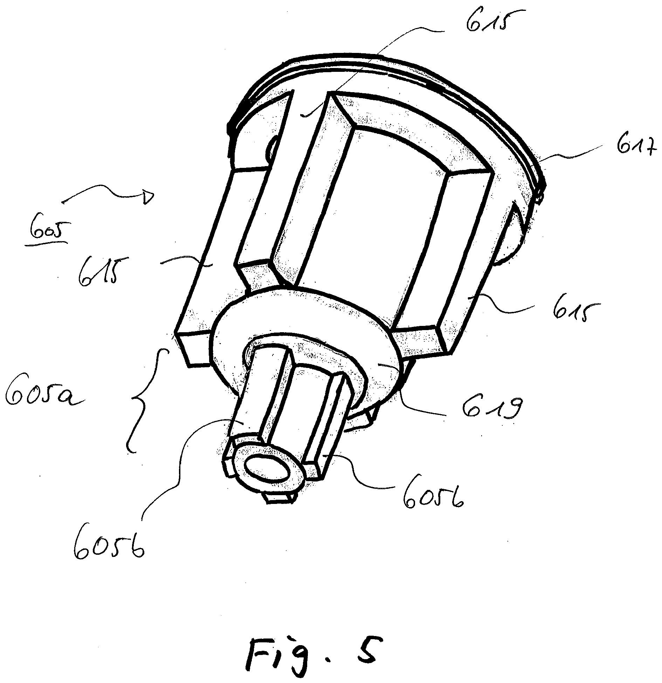

In Fig. 3 nimmt die Verschlussvorrichtung 605 die erste Stellung ein. Die Sterilisationsöffnung 601 wird daher nicht durch die hier rein beispielhaft als Verschlussstopfen ausgestaltete Verschlussvorrichtung 605 fluiddicht verschlossen. Sie bleibt vielmehr offen oder zumindest teilweise offen, da die Verschlussvorrichtung 605 auch in einem unteren (bezogen auf die Darstellung der Fig. 5), gegenüber einem oberen Abschnitt der Verschlussvorrichtung 605 verjüngten (also einen kleineren Durchmesser aufweisenden) Abschnitt 605a Streben 605b (hier exemplarisch vier Stück; eine andere Anzahl an Streben 605b ist ebenfalls von der vorliegenden Erfindung umfasst) aufweist, siehe Fig. 5. Fluid kann zwischen ihnen hindurch oder an ihnen vorbei in das Innere der Kassette 1000 eindringen. In der in Fig. 4 gezeigten Stellung ist dies hiervon abweichend anders.

Die Verschlussvorrichtung 605 kann, wie in Fig. 3 gezeigt, rein exemplarisch eine Verrastvorrichtung 607 aufweisen. Letztere ist vorgesehen, um die Verschlussvorrichtung 605 in der zweiten, geschlossenen Stellung, in welcher Fluid nicht über die Sterilisationsöffnung 601 in die Kassette 1000 eindringen kann, zu halten, sobald ein Verrasten stattgefunden hat.

In Fig. 3 ist die Verschlussvorrichtung 605 allerdings nicht mittels der Verrastvorrichtung 607 verrastet, und die Sterilisationsöffnung 601 ist folglich in der ersten, geöffneten Stellung gezeigt.

**Fig. 4** zeigt die Sterilisationsöffnung 601 in der zweiten, geschlossenen Stellung. Die rein optional vorhandene Verrastvorrichtung 607 ist verrastet. Hierzu greift, wie hier exemplarisch gezeigt, wenigstens ein elastisches oder nicht elastisches Element, welches beispielsweise kreisförmig oder mit Unterbrechungen um einen Umfang angeordnet ist, in eine, beispielsweise kreisförmig verlaufende, Vertiefung der Verschlussvorrichtung 605 ein. Alternativ oder ergänzend hintergreift ein elastischer Vorsprung 609 der Verschlussvorrichtung 605 kreisförmig oder auf andere Weise einen Abschnitt des Hartteils 1, beispielsweise eine Öffnung der Sterilisationsöffnung 601, oder verspreizt sich hinter dieser.

Für den Fachmann ist erkennbar, dass die vorliegende Erfindung auch Ausgestaltungen umfasst, bei welcher das Hartteil 1 einen elastischen Vorsprung hat, welcher einen Abschnitt der Verschlussvorrichtung 605 hintergreift oder sich hinter diesem verspreizt.

Die Verrastung stellt vorteilhaft sicher, dass sich die Verschlussvorrichtung 605 nicht ungewollt oder unbeabsichtigt von der zweiten Stellung zurück in die erste Stellung bewegt. Dies kann vorteilhaft der Sicherheit bei der Benutzung der Kassette 1000 dienen.

Das Überführen der Verschlussvorrichtung 605 in die zweite Stellung bewirkt, dass wenigstens eine erste Dichtflächenpaarung 611 und/oder, optional, auch eine zweite Dichtflächenpaarung 613 in Kontakt oder in Eingriff miteinander kommen. Durch diesen Kontakt oder Eingriff wird eine Abdichtung der Verschlussvorrichtung 605 gegenüber dem Hartteil 1, hier exemplarisch gegenüber der Verrastvorrichtung 607 einerseits und gegenüber dem Zylinderabschnitt 603 andererseits, bewirkt.

**Fig. 5** zeigt die Verschlussvorrichtung 605 der Fig. 3 und 4 in perspektivischer Ansicht.

Zu erkennen ist, dass die Verschlussvorrichtung 605 entlang ihres Umfangs mit in Längserstreckung (also in einer Richtung senkrecht jener Ebene, in welcher der Umfang liegt) der Verschlussvorrichtung 605 verlaufenden Streben 615 ausgestaltet ist. Zwischen ihnen hindurch, oder an ihnen vorbei oder entlang, ist eine Durchlässigkeit für das Sterilisationsfluid gegeben. Die Durchlässigkeit ist dann aufgehoben oder unterbunden, wenn ein oberer Ringabschnitt 617 und/oder ein unterer Ringabschnitt 619 geeignet an Abschnitten des Hartteils 1 zum Anliegen kommen, wie in Fig. 4 gezeigt.

Die Verschlussvorrichtung 605 kann einen elastischen Vorsprung 609 aufweisen, wie in Fig. 4 gezeigt, auch wenn dieser in Fig. 5 nicht dargestellt ist. Ein solcher Vorsprung 609 ist jedoch optional.

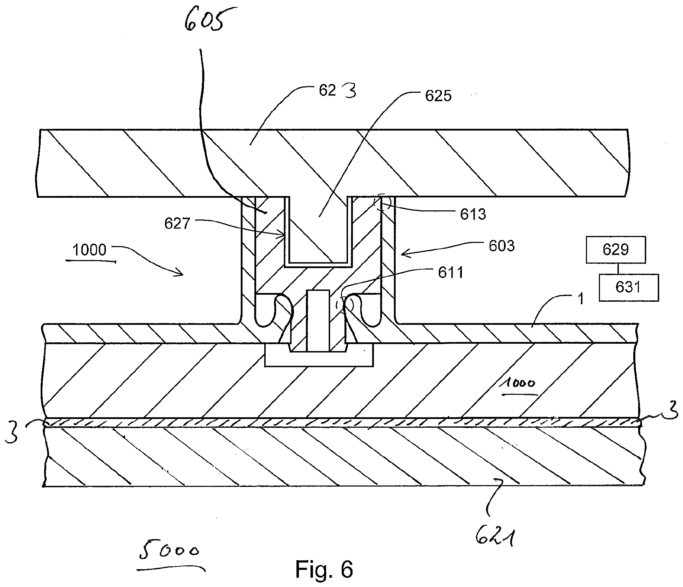

**Fig. 6** zeigt eine Aktor-Sensor-Matte 621 einer Aktor-SensorEinheit und eine Maschinentür 623 eines schematisch vereinfachten Ausschnitts einer erfindungsgemäßen Blutbehandlungsvorrichtung 5000.

Zwischen Aktor-Sensor-Matte 621 und Maschinentür 623 ist eine nur durch ihr Hartteil 1 mit der Sterilisationsöffnung 601, ihren Zylinderabschnitt 603 und ihre Verschlussvorrichtung 605 angedeutete Kassette 1000 verpresst.

Ein mit der Maschinentür 623 verbundener oder durch diese hindurch gesteckter Aktuator oder Aktor 625 ist in eine Vertiefung oder einen Eingriff 627 der Verschlussvorrichtung 605 eingeführt. Es ist im Beispiel der Fig. 6 der Aktuator 625, der die Verschlussvorrichtung 605 in die in Fig. 6 gezeigte zweite, geschlossene Stellung übergeführt hat.

Im Beispiel der Fig. 6 ist der Aktuator 625 passiv, d. h. nicht mittels einer Hydraulik, Mechanik oder auf andere Weise in den Eingriff 627 aktiv einführbar. Vielmehr wird er, jedenfalls was den Aktuator 625 als solchen angeht, durch Schließen der Maschinentür 623 ausreichend tief in den Eingriff 627 gebracht bzw. die Kassette 1000 wird durch Schließen der Maschinentür 623 ausreichend weit auf ihn aufgeschoben, was hierin zur Abgrenzung gegenüber einem Aktuator, welcher mittels Elektromechanik, Pneumatik, Mechanik oder auf andere Weise relativ zu seiner unmittelbaren Umgebung, etwa der Maschinentür oder der Aktuator-Sensor-Matte, mit welcher er jeweils verbunden ist, verschoben wird, als passiv bezeichnet wird.

Die Sterilisationsöffnung 601 ist in Fig. 6 ebenso wie in Fig. 4, aber anders als in Fig. 3, verschlossen und daher in Fig. 4 oder Fig. 6 weder als "Öffnung" im eigentlichen Wortsinn erkennbar noch mit Bezugszeichen versehen. Die Lage der Sterilisationsöffnung 601 kann - beispielsweise bei Vergleich mit der Fig. 3 - allerdings auch für die Fig. 4 oder die Fig. 6 ohne Mühe bestimmt werden.

Ein Sensor 629 kann vorgesehen sein, um einen erfolgten Übergang der Verschlussvorrichtung 605 in die zweite Stellung zu dokumentieren. Zu diesem Zweck kann der Sensor 629 als Bewegungsfühler, Tastsensor, Drucksensor, optischer Sensor oder dergleichen ausgestaltet und eingesetzt werden.

Ein entsprechendes Signal des Sensors 629 kann einer Steuervorrichtung 631 der Blutbehandlungsvorrichtung 5000 zugeführt werden. Sie kann programmiert oder konfiguriert sein, um ein Behandeln eines Patienten nur dann mittels der Blutbehandlungsvorrichtung 5000 zuzulassen, wenn mittels Sensor 629 festgestellt wurde, dass die Verschlussvorrichtung 605 in die zweite Stellung verbracht wurde, jedenfalls aber nicht mehr in der ersten Stellung steht.

### Bezugszeichenliste

| **Bezugszeichen** | **Beschreibung** |
|---|---|
| 1000 | Blutbehandlungskassette oder Kassette |
| 1 | Hartteil |
| 3 | Folie |
| 4 | Dichtsteg |
| 5 | umlaufende Schweißnaht |
| 7 | arterieller Patientenanschluss |
| 9 | arterielle Druckmesskammer |
| 11 | Konnektor für Blutaustritt aus Kassette 1000 |
| 13 | Konnektor für Bluteintritt in Kassette 1000 |
| 15 | Kammer mit arterieller Post-Pumpe- bzw. Präfilter-Druckmessstelle |
| 17 | arterielle Filterleitung |
| 19 | venöse Filterleitung |
| 21 | venöse Blutkammer |
| 23 | oberer Raum der venösen Blutkammer 21 |
| 25 | unterer Raum der venösen Blutkammer 21 |
| 27 | Querschnittsverjüngung des Hartteils 1 |
| 29 | Gerinnselfänger |
| 31 | venöser Patientenanschluss |
| 33 | arterielle Heparin-Zugabestelle |
| 35 | Rückschlagventil der arteriellen Heparin-Zugabestelle 33 |
| 36 | arterielles Heparin-Zugabeventil (Phantomventil) |
| 37 | venöse Heparin-Zugabestelle |
| 39 | Rückschlagventil der venösen Heparin-Zugabestelle 37 |
| 40 | venöses Heparin-Zugabeventil (Phantomventil) |
| 41 | Substituat-Zugabestelle |
| 43 | Konnektor für Substituataustritt aus der Kassette 1000 |
| 45 | Konnektor für Substituateintritt in die Kassette 1000 |
| 47 | Rückschlagventil für Substituatzugabe |
| 49 | Substituatleitung |
| 51 | Prädilutions-Zugabeventil (Phantomventil) |
| 53 | Postdilutions-Zugabeventil (Phantomventil) |
| 55 | Single-Needle-Sterilmembran |
| 57 | Single-Needle-Kammer |
| 59 | Blutschwallumleitungselement |
| 61 | Single-Needle-Blutventil (Phantomventil) |
| 63 | Absaugstelle für Vakuumankopplung |
| 65 | primäres Ausrichtungszentrum |
| 67 | sekundäre Ausrichtungsstelle |
| 69 | Dichtsteg |
| 90 | Pumpschlauchsegment der Substituatpumpe |
| 93 | venöser Abschnitt des extrakorporalen Kreislaufs |
| 601 | Sterilisationsöffnung |
| 603 | Zylinderabschnitt |
| 605 | Verschlussvorrichtung |
| 605a | verjüngter Abschnitt |
| 605b | Streben |
| 607 | Verrastvorrichtung |
| 609 | Vorsprung |
| 611 | Dichtflächenpaarung oder Dichtelement |
| 613 | Dichtflächenpaarung oder Dichtelement |
| 615 | Streben |
| 617 | oberer Ringabschnitt |
| 619 | unterer Ringabschnitt |
| 621 | Aktor-Sensor-Matte |
| 623 | Maschinentür |
| 625 | Aktuator oder Aktor |
| 627 | Eingriff |
| 629 | Sensor |
| 631 | Steuervorrichtung |
| 5000 | Maschine oder Blutbehandlungsvorrichtung |

## Patentansprüche

1. Blutbehandlungskassette (1000) mit einem als Hartteil (1) ausgestalteten Kassettenkörper und optional einer Folie (3), welche mit dem Hartteil (1) verbunden ist und das Hartteil (1) zumindest abschnittsweise abdeckt,
wobei die Blutbehandlungskassette (1000) oder ihr Hartteil (1) wenigstens eine Sterilisationsöffnung (601) und wenigstens eine Verschlussvorrichtung (605) für die Sterilisationsöffnung (601) aufweist,
wobei die Verschlussvorrichtung (605) ausgestaltet ist, um in einem Zustand, in welchem sie mit der Sterilisationsöffnung (601) verbunden ist, von einer ersten Stellung, in welcher sie einen Fluidaustausch zwischen einem Inneren und einem Äußeren der Blutbehandlungskassette (1000) mittels der Sterilisationsöffnung (601) zulässt, in eine zweite Stellung, in welcher sie einen Fluidaustausch zwischen dem Inneren und dem Äußeren der Blutbehandlungskassette (1000) mittels der Sterilisationsöffnung (601) nicht zulässt, überführbar zu sein,
**gekennzeichnet dadurch, dass** die Verschlussvorrichtung (605) mit einem Mechanismus ausgestaltet ist, mittels welchem die Verschlussvorrichtung (605) in der ersten und in der zweiten Stellung gehalten werden kann.

2. Blutbehandlungskassette (1000) nach Anspruch 1, wobei der Mechanismus eine Verrastvorrichtung (607) ist oder eine solche aufweist.

3. Blutbehandlungskassette (1000) nach einem der vorangegangenen Ansprüche, wobei die Verschlussvorrichtung (605) wenigstens zwei Dichtflächenpaarungen (611, 613), Dichtabschnitte oder Dichtelemente aufweist.

4. Blutbehandlungsvorrichtung (5000), mit einer Blutbehandlungskassette (1000) nach einem der vorangegangenen Ansprüche, mit wenigstens einem Aktuator (625) versehen, welcher ausgestaltet und/oder konfiguriert ist für eine Interaktion mit der Verschlussvorrichtung (605) der Blutbehandlungskassette (1000), um die Verschlussvorrichtung (605) von der ersten Stellung in die zweite Stellung überzuführen.

5. Blutbehandlungsvorrichtung (5000) nach Anspruch 4, wobei der Aktuator (625) eine unveränderbare Erhebung in oder an einer Aktor-Sensor-Matte (621), einer Maschinentür (623) oder in oder an einer Aktor-Sensor-Platte ist.

6. Blutbehandlungsvorrichtung (5000) nach einem der Ansprüche 4 bis 5, welche eine Vorrichtung aufweist, welche konfiguriert ist, den Aktuator (625) oder eine seiner Eigenschaften zu verändern und/oder die Verschlussvorrichtung (605) mittels des Aktuators (625) von der ersten Stellung in die zweite Stellung zu überführen.

7. Blutbehandlungsvorrichtung (5000) nach einem der Ansprüche 4 bis 6, mit einer Steuervorrichtung (631) und wenigstens einem Sensor (629),
wobei die Blutbehandlungsvorrichtung (5000) konfiguriert ist, um mittels des Sensors (629) festzustellen, ob sich die Verschlussvorrichtung (605) in der zweiten Stellung befindet, und
wobei die Blutbehandlungsvorrichtung (5000) ferner konfiguriert ist, eine Blutbehandlung mittels der Blutbehandlungsvorrichtung (5000) zu unterbinden oder zu unterbrechen, sollte festgestellt worden sein, dass sich die Verschlussvorrichtung (605) nicht in der zweiten Stellung befindet.

## Claims

1. A blood treatment cassette (1000) having a cassette body embodied as a hard part (1) and having optionally a film (3) connected to the hard part (1) and at least partially covering the hard part (1),
the blood treatment cassette (1000) or its hard part (1) comprising at least one sterilization opening (601) and at least one closure device (605) for the sterilization opening (601),
wherein the closure device (605) is embodied, when being in a state connected to the sterilization opening (601), to be transferable from a first position in which it allows a fluid exchange between an interior and an exterior of the blood cassette (1000) by means of the sterilization opening (601), into a second position in which it does not allow a fluid exchange between the interior and the exterior of the blood treatment cassette (1000) by means of the sterilization opening (601),
**characterized in that**
the closure device (605) comprises a mechanism,
by means of which the closure device (605) may be maintained in the first and in the second position.

2. The blood treatment cassette (1000) according to claim 1, wherein the mechanism is, or comprises, a latching device (607).

3. The blood treatment cassette (1000) according to anyone of the preceding claims, wherein the closure device (605) comprises at least two pairs of sealing surfaces (611, 613), sealing sections or sealing elements.

4. A blood treatment apparatus (5000) comprising a blood treatment cassette (1000) according to anyone of the preceding claims, provided with at least one actuator (625) embodied and/or configured to interact with the closure device (605) of the blood treatment cassette (1000) in order to transfer the closure device (605) from the first position into the second position.

5. The blood treatment apparatus (5000) according to claim 4, wherein the actuator (625) is an immutable protrusion in or on an actuator-sensor mat (621), a machine door (623) or in or on an actuator-sensor plate.

6. The blood treatment apparatus (5000) according to anyone of claims 4 to 5, comprising a device configured to change the actuator (625) or one of its properties and/or to transfer the closure device (605) from the first position into the second position by means of the actuator (625).

7. The blood treatment apparatus (5000) according to anyone of claims 4 to 6, comprising a control device (631) and at least a sensor (629),
wherein the blood treatment apparatus (5000) is configured to determine by means of the sensor (629) whether the closure device (605) is in the second position, and
wherein the blood treatment apparatus (5000) is further configured to prevent or interrupt a blood treatment by means of the blood treatment apparatus (5000), should it be determined that the closure device (605) is not in the second position.

## Revendications

1. Une cassette de traitement du sang (1000) comprenant un corps de cassette conçu comme une partie rigide (1) et comprenant optionnellement un film (3) relié à la partie rigide (1) et recouvrant au moins partiellement la partie rigide (1),
la cassette de traitement du sang (1000) ou sa partie rigide (1) comprenant au moins une ouverture de stérilisation (601) et au moins un système de fermeture (605) pour l'ouverture de stérilisation (601),
où le système de fermeture (605) est conçu, lorsqu'il est dans un état de connexion avec l'ouverture de stérilisation (601), pour être transférable d'une première position, permettant un échange de fluide entre un intérieur et un extérieur de la cassette de traitement du sang (1000) au moyen de l'ouverture de stérilisation (601), à une seconde position ne permettant pas d'échange de fluide entre l'intérieur et l'extérieur de la cassette de traitement du sang (1000) au moyen de l'ouverture de stérilisation (601),
**caractérisé en ce que**
le système de fermeture (605) est équipé d'un mécanisme, au moyen duquel le système de fermeture (605) peut être maintenu dans la première et dans la seconde position.

2. La cassette de traitement du sang (1000) selon la première revendication, où le mécanisme est, ou comprend, un dispositif d'enclenchement (607).

3. La cassette de traitement du sang (1000) selon l'une quelconque des revendications précédentes, où le système de fermeture (605) comprend au moins deux paires de surfaces d'étanchéité (611, 613), des sections d'étanchéité ou des éléments d'étanchéité.

4. Un appareil de traitement du sang (5000) comprenant une cassette de traitement du sang (1000) selon l'une quelconque des revendications précédentes, muni d'au moins un actionneur (625) conçu et/ou configuré pour interagir avec le système de fermeture (605) de la cassette de traitement du sang (1000) afin de transférer le système de fermeture (605) de la première position à la seconde position.

5. L'appareil de traitement du sang (5000) selon la revendication 4, où l'actionneur (625) est une saillie immuable dans ou sur un tapis actionneur-capteur (621), une porte de machine (623) ou dans ou sur une plaque actionneur-capteur.

6. L'appareil de traitement du sang (5000) selon l'une quelconque des revendications 4 à 5, comprenant un dispositif configuré pour modifier l'actionneur (625) ou une de ses propriétés et/ou pour transférer le système de fermeture (605) de la première position à la seconde position au moyen de l'actionneur (625).

7. L'appareil de traitement du sang (5000) selon l'une quelconque des revendications 4 à 6, comprenant un dispositif de commande (631) et au moins un capteur (629),
où l'appareil de traitement du sang (5000) est configuré pour déterminer à l'aide du capteur (629) si le système de fermeture (605) se trouve dans la seconde position, et
où l'appareil de traitement du sang (5000) est en outre configuré pour empêcher ou interrompre un traitement du sang au moyen de l'appareil de traitement du sang (5000), s'il a été déterminé que le système de fermeture (605) n'est pas dans la seconde position.
